# EUROPEAN PATENT APPLICATION

(11) **EP 4 570 168 A1**
(43) Date of publication of application: **18.06.2025**
(21) Application number: 24218917.3
(22) Date of filing: 11.12.2024
(51) Int. Cl.: A61B 5/00, A61B 5/367, A61B 5/363, A61B 5/361, A61B 5/349, A61B 5/318, A61B 18/00

(54) **GUI TO VISUALLY CONNECT FEATURES IDENTIFIED IN ELECTROANATOMICAL (EA) MAP TO ECG SIGNALS**

(30) Priority: 12.12.2023 US 202318536357
(71) Applicant: Biosense Webster (Israel) Ltd., 2066717 Yokneam (IL)
(72) Inventor: GOVARI, Assaf, 2066717 Yokneam (IL); ALTMANN, Andres Claudio, 2066717 Yokneam (IL)
(74) Representative: Carpmaels & Ransford LLP

(57) **Abstract**

A system includes a display device and a processor. The processor is configured to (i) present, on the display device, intracardiac electrograms recorded over tissue of a portion of a cardiac chamber, (ii) identify a subset of the electrograms that show arrhythmogenic activity, (iii) analyze one or more characteristics of the arrhythmogenic activity, and (iv) graphically interconnect the electrograms in the subset to present the one or more characteristics of the arrhythmogenic activity to a user.

## Description

### FIELD OF THE DISCLOSURE

This disclosure relates generally to the diagnosis and treatment of cardiac arrhythmias, and particularly, to graphically indicating intracardiac electrograms that correspond together to a regional arrhythmogenic electrical activity in a cardiac chamber.

### BACKGROUND OF THE DISCLOSURE

Displaying intracardiac electrograms acquired using a multi-electrode catheter including presenting spatiotemporal analyses of these was previously proposed in the patent literature. For example, U.S. Patent 10,349,855 describes recording intracardiac electrograms using a multi-electrode catheter and establishing respective annotations. Within a time window, a pattern comprising a monotonically increasing local activation time sequence from a set of electrograms from neighboring electrodes is detected. The set is reordered and displayed for the operator.

As another example, U.S. Patent Application Publication 2022/0369991 describes medical apparatus and methods for diagnostic and site determination of cardiac arrhythmias within the heart of a subject. A computing device receives, records, and processes electrocardiogram (ECG) signals in the form of bipolar and unipolar ECGs associated with respective cardiac tissue locations corresponding to catheter distal end sensors on locations. Unipolar ECGs that include signals from a plurality of successive heartbeats corresponding to locations within an area of study are analyzed to identify Fractionated Unipolar ECG Signal Complexes (FUESCs) of unipolar ECGs by defining complexes of the unipolar ECGs that correspond to respective bipolar activity windows.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a catheter-based electroanatomical (EA) mapping and ablation system, in accordance with an example of the present disclosure;
Fig. 2A shows a graphical indication on an EA map of a focal source type of arrhythmia graphically tied to a subset of intracardiac electrograms, the subset collectively graphically indicated to assess the arrhythmia, in accordance with an example of the present disclosure;
Fig. 2B shows a graphical indication on an EA map of a rotor type of arrhythmia graphically tied to a subset of intracardiac electrograms, the subset collectively graphically indicated to assess the arrhythmia, in accordance with an example of the present disclosure;
Fig. 3 is a rendering of an EA map superimposed with regions of interest demonstrating possible arrhythmia and respective intracardiac electrograms from that regions, in accordance with an example of the present disclosure; and
Fig. 4 is a flow chart that schematically illustrates a method to tie a graphical indication of a regional arrhythmogenic activity on an EA map to a subset of intracardiac electrograms, and collectively graphically indicated on the subset of arrhythmogenic characteristics, in accordance with an example of the present disclosure.

### DETAILED DESCRIPTION OF EXAMPLES

### OVERVIEW

Cardiac arrhythmias, such as atrial fibrillation, are a group of conditions in which the heart beats with an irregular rhythm. Electro-anatomical (EA) mapping of a patient's heart may serve as the basis for deciding on a therapeutic course of action, such as tissue ablation, to restore normal heart rhythm by altering the propagation of electrical activity in heart tissue.

Electrical activity at a tissue region in the heart may be measured by contacting the tissue with electrodes of a catheter having a location sensor in its distal end and, simultaneously, acquiring intracardiac electrograms at respectively measured locations in the region. Optionally, the analysis may be performed based on data accumulated over time. The acquired data points are used to generate a detailed cardiac EA map of diagnostic value. Using EA mapping, electrical properties of heart tissue, such as local activation time (LAT) and local activation amplitude, may be visualized over a rendering of a portion of the heart.

Many electrophysiologists, however, prefer to inspect, in detail, the intracardiac electrograms themselves in order to assess the regional arrhythmogenic characteristics (e.g., change in amplitude and/or timing of activations, correspondences between annotated activations on the same or different electrograms, and changing patterns, such as fractionations, in activations). Yet, practical experience has shown the difficulty for electrophysiologists to take in the information provided by both the EA map and the intracardiac electrograms. Moreover, a majority of electrograms (out of as much as of a hundred electrograms presented) at each given acquisition by a multi-electrode catheter may be irrelevant, making it difficult to use the electrograms alone.

Examples of the present disclosure that are described herein provide a technique to graphically emphasize, on a display, a subset of the intracardiac electrograms that may best illustrate characteristics of a regional arrhythmia. The subset is automatically identified by a processor based on analyzing the instant electro-cardiac signals captured and/or based on analysis of the EA map. An algorithm to identify the subset typically does so by detecting some spatiotemporal relationships among the relevant electrograms.

In one example, a processor uses an algorithm to graphically point from the regional arrhythmia displayed on the EA map (e.g., of a focal source or rotor circle of arrhythmia) to electrograms of the subset. To this end, the processor, or the user, initially identifies and marks the regional arrhythmogenic tissue on the EA map. The disclosed method may graphically present the connection between the region in the EA map and the subset using arrows, annotations, highlighting, and other methods.

The processor runs an algorithm that analyzes the subset to assess the characteristics of the arrhythmogenic activity. Such characteristics may include at least one of the activation amplitudes, activation timings, and activation patterns (e.g., electrogram signal fractionation). The processor then graphically interconnects the electrograms in the subset in a way that visually emphasizes, on the subset, the characteristics (e.g., propagation) of arrhythmogenic activity to a user.

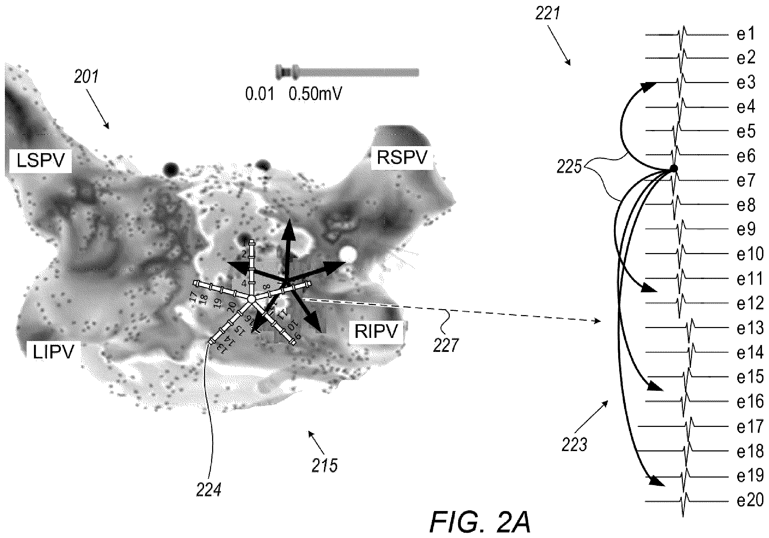

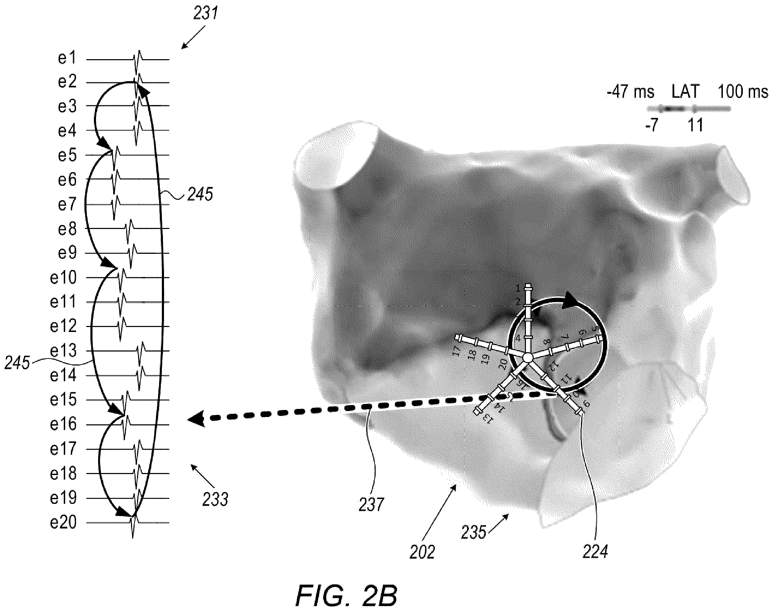

In known systems, the electrodes at the distal end of the catheter are associated with numbers and the electrograms sensed from the electrodes are typically displayed in real time in number order, as seen in Fig. 2. This may assist a user in understanding a spatiotemporal relationships among the relevant electrograms. In some example systems, up to 100 electrodes may simultaneously collect an electrogram signal and all the electrograms may be displayed in numerical order alongside an EA map that is being constructed.

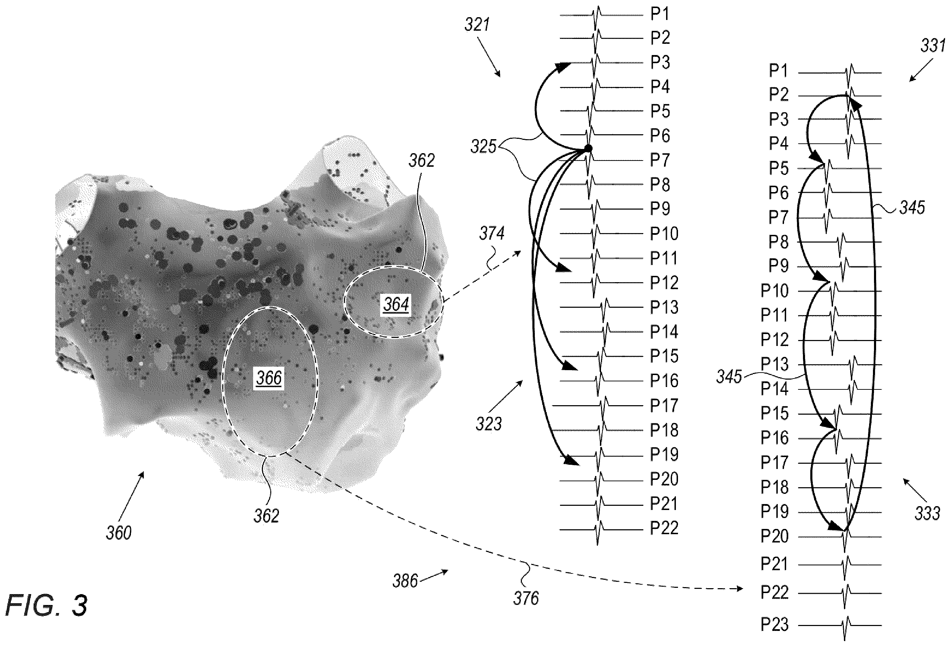

Electrograms may also be displayed while viewing an existing, e.g., stored EA map. Electrograms over a selected region of interest may be displayed alongside the EA map. The order at which the electrograms are displayed may follow a numerical order of points collected in the region of interest, as seen in Fig. 3. Each electrogram is associated with a location at which it was captured.

A propagation along the tissue may not necessarily follow the order at which the electrograms are displayed. In such cases it may be difficult for a user to follow the propagation when viewing the electrograms. For example, it may be difficult to follow a local focal or rotary propagation that appears in only a portion of the electrograms. In examples of the present disclosure, a spatiotemporal analysis of the electrograms is performed to detect a pattern of progression in an area of interest. Based on the analysis, a progression of arrows between respective electrograms is added to indicate the progression of a detected propagation (e.g., Fig. 2). Optionally, the processor is configured based on user selection, to selectively display a subset of the electrograms associated with the detected propagation signal. Optionally, the processor is also configured based on user selection, to rearrange the order at which the subset is displayed so that it corresponds to the order of the detected propagation.

The processor provides graphical indications (e.g., arrows) that show the behavior (e.g., progression) of the arrhythmia on the electrograms. As each electrogram is acquired by an electrode at some tissue location, the user may infer a spatial order from the order of the electrograms.

In some examples, the processor graphically interconnects the electrograms based on a spatiotemporal relation between arrhythmogenic activations in the electrograms. To this end, the processor annotates arrhythmogenic activations of the arrhythmogenic activity over at least one of the electrograms in the subset.

As noted above, the processor may use arrows to graphically interconnect the electrograms of the subset, e.g., to show a progression of an aberrant electrical activity. In another example, the processor graphically highlights at least one electrogram in the subset (e.g., one that serves as a healthy reference location or a critically ill location).

### SYSTEM DESCRIPTION

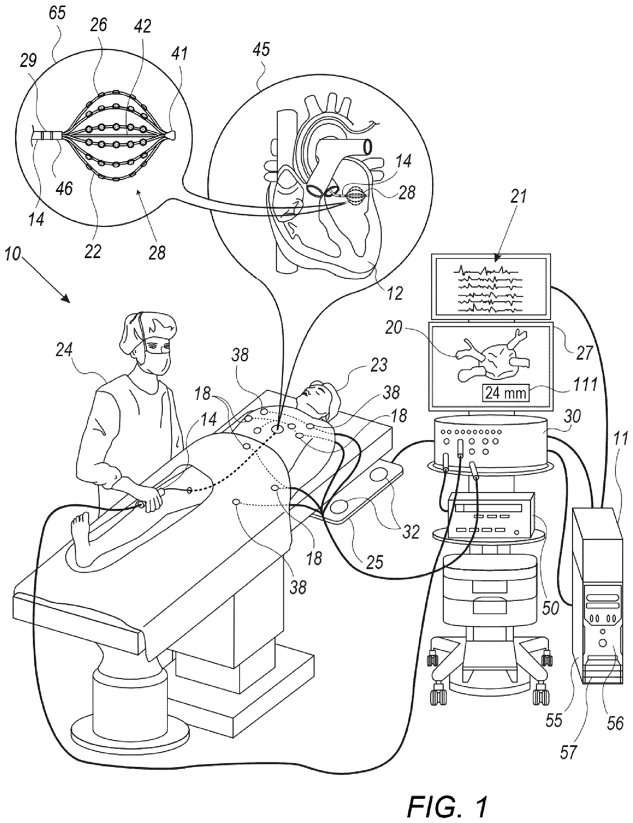

Fig. 1 is a schematic, pictorial illustration of a catheter-based electro-anatomical (EA) mapping and ablation system 10, in accordance with an example of the present disclosure.

System 10 includes multiple catheters which are percutaneously inserted by physician 24 through the patient's vascular system into a chamber or vascular structure of a heart 12 (seen in inset 45). Typically, a delivery sheath catheter is inserted into a cardiac chamber, such as the left or right atrium near a desired location in heart 12. Thereafter, a plurality of catheters is inserted into the delivery sheath catheter in order to arrive at the desired location. The plurality of catheters may include a catheter dedicated for pacing, a catheter for sensing intracardiac electrogram signals, a catheter dedicated for ablating and/or a catheter dedicated for both EA mapping and ablating. An example catheter 14, illustrated herein, is configured for sensing bipolar electrograms. Physician 24 brings a distal tip 28 (also called hereinafter distal end assembly 28) of catheter 14 into contact with the heart wall for sensing a target site in heart 12. For ablation, physician 24 similarly brings a distal end of an ablation catheter to a target site.

As seen in inset 65, catheter 14 is an exemplary catheter that includes a basket distal end 28, including one, and preferably multiple, electrodes 26 optionally distributed over a plurality of splines 22 at distal tip 28 and configured to sense IEGM signals. Catheter 14 may additionally include a position sensor 29 embedded in or near distal tip 28 on a shaft 46 of catheter 14, used to track the position and orientation of distal tip 28. Optionally, and preferably, position sensor 29 is a magnetic-based position sensor including three magnetic coils for sensing three-dimensional (3D) position and orientation. As seen, distal tip 28 further includes an expansion/collapse rod 42 of expandable assembly 28 that is mechanically connected to basket assembly 28 at a distal edge 41 of assembly 28.

Magnetic based position sensor 29 may be operated together with a location pad 25 that includes a plurality of magnetic coils 32 configured to generate magnetic fields in a predefined working volume. Real-time position of distal tip 28 of catheter 14 may be tracked based on magnetic fields generated with location pad 25 and sensed by magnetic based position sensor 29. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish a location reference for location pad 25 as well as impedance-based tracking of electrodes 26. For impedance-based tracking, electrical current is directed toward electrodes 26 and sensed at electrode skin patches 38 so that the location of each electrode can be triangulated via electrode patches 38. Details of the impedance-based location tracking technology are described in US Patent Nos. 7,536,218; 7,756,576; 7,848,787; 7,869,865; and 8,456,182.

A recorder 11 displays, on display device 27, cardiac signals 21 (e.g., electrograms acquired at respectively tracked cardiac tissue positions) acquired with body surface ECG electrodes 18 and intracardiac electrograms acquired with electrodes 26 of catheter 14. Recorder 11 may include pacing capability to pace the heart rhythm, and/or may be electrically connected to a standalone pacer.

Workstation 55 includes memory 57, a processor 56 unit with memory or storage with appropriate operating software loaded therein, and user interface capability. Workstation 55 may provide multiple functions, optionally including (i) modeling endocardial anatomy in three-dimensions (3D) and rendering the model or EA map 20 for display on display device 27, (ii) displaying on display device 27 activation sequences (or other data) compiled from recorded cardiac signals 21 in representative visual indicia or imagery superimposed on the rendered EA map 20, (iii) displaying real-time location and orientation of multiple catheters within the heart chamber, and (iv) displaying sites of interest on display device 27, such as places where ablation energy has been applied. One commercial product embodying elements of system 10 is available as the CARTO^{™} 3 System, available from Biosense Webster, Inc., 31A Technology Drive, Irvine, CA 92618.

In a disclosed example, processor 56 runs an algorithm that identifies a subset of electrograms 21 that shows arrhythmogenic activity. The processor analyzes a type of arrhythmogenic activity and graphically interconnects the electrograms in the subset to present the type of arrhythmogenic activity to a user. Processor 56 uses the algorithm to graphically tie intracardiac-electrogram-based analysis with a graphical indication on EA map 20. Physician 24 can select/unselect, e.g., via a graphical user interface (GUI) 111, how to operate the algorithm and/or the graphics.

System 10 may include an ablation energy generator 50 that is adapted to conduct ablative energy to one or more electrodes at a distal tip of a catheter configured for ablation. Energy produced by ablation energy generator 50 may include, but is not limited to, radiofrequency (RF) energy or pulsed-field ablation (PFA) energy, including monopolar or bipolar high-voltage DC pulses, to be used to effect irreversible electroporation (IRE), or combinations thereof.

Patient interface unit (PIU) 30 is an interface configured to establish electrical communication between catheters, electrophysiological equipment, power supply and a workstation 55 to control system 10 operation and to receive EA signals from the catheter. Electrophysiological equipment of system 10 may include, for example, multiple catheters, location pad 25, body surface ECG electrodes 18, electrode patches 38, ablation energy generator 50, and recorder 11. Optionally, and preferably, PIU 30 additionally includes processing capability for implementing real-time computations of catheter locations and for performing ECG calculations.

In some examples, processor 56 typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

This configuration of system 10 is shown by way of example, to illustrate certain problems that are addressed by examples of the present disclosure and to demonstrate the application of these examples in enhancing the performance of such a system. Examples of the present disclosure, however, are by no means limited to this specific sort of example system, and the principles described herein may similarly be applied to other types of medical systems. For example, other multi-electrode catheter types may be used, such as the multi-arm OCTARAY^{™} catheter or a flat catheter.

### GUI TO VISUALLY CONNECT FEATURES IDENTIFIED IN THE EA MAP TO ECG SIGNALS

In Fig. 2, a multi arm mapping catheter is schematically shown superimposed (224) on the EA maps) was used in acquiring the electrograms. Spatiotemporal relationships may be found among the relevant electrograms by numbering the electrograms according to the electrode numbers (in the shown case there are 20 electrodes, four on each arm, and the electrograms 221 in Fig. 2A and 231 in Fig. 2B are accordingly numbered e1, e2, ... e20) . The electrograms that are showed is a schematic representation of signals obtained from the example mapping catheter 224.

Fig. 2A shows a graphical indication (215) on an EA map (201) of a focal source type of arrhythmia graphically tied (227) to a subset (223) of intracardiac electrograms (221), the subset (223) graphically indicated by arrows 225 to assess the spatiotemporal progression of the focal arrhythmia, in accordance with an example of the present disclosure.

Fig. 2B shows a graphical indication (235) on an EA map (202) of a rotor type of arrhythmia graphically tied (237) to a subset (233) of intracardiac electrograms (231), the subset (233) graphically indicated by arrows 245 to assess the spatiotemporal progression of the rotor arrhythmia, in accordance with an example of the present disclosure.

EA maps 201 and 202 both comprise a rendering of an anatomical surface of a cardiac chamber (e.g., a left atrium) and respective electrical values (e.g., activation amplitudes and/or times) superimposed on the rendered anatomical surface at each mapped location.

Processor 56 of system 10 presents, on the display device 27, intracardiac electrograms (221, 231) recorded over tissue of a portion of a cardiac chamber shown by EA maps 201 and 202. Using the EA map, the processor identifies a subset of the electrograms that show arrhythmogenic activity.

The processor analyzes some characteristics of the arrhythmogenic activity and graphically interconnects (225, 245) the electrograms in the subset to present the characteristics of the arrhythmogenic activity to a user. The characteristics may comprise activation amplitudes, activation timings, and activation patterns.

As seen, the processor graphically indicates the type of arrhythmogenic activity on the EA map (e.g., by indicating a regional focal source type (215) or a rotor type of arrhythmogenic activity (235)). The processor graphically connects (227, 237) the activity on the EA map to at least one of the electrograms in the respective subset (223, 233).

As an option, a user can manually indicate some of the activity described above instead of the processor's automatic indications. For example, the user may identify the regional focal source or rotor types of arrhythmogenic activity on the EA map.

Typically, the algorithm run by the processor annotates arrhythmogenic activations of the arrhythmogenic activity over at least one of the electrograms in the subset.

Fig. 3 is a rendering of an EA map 360 superimposed with regions of interest 362 demonstrating possible arrhythmia and respective schematically presented intracardiac electrograms (321, 331) from that regions, in accordance with an example of the present disclosure. The region are defined by schematically shown encirclements that a processor or a user may apply to the map and include data points (364, 366) comprising each a recorded electrode position and a recorded electrogram at the position. In this case the order is based on the numbering of the points on the map. It can be collected over time. The electrograms (321, 331) are numbered according to the data points index in each regions as P1, P2, ...PN.

In the shown example a user views an existing EA map. When viewing the map the user can select (362) a region and the processor displays to the user all the electrograms (321, 3331) in the selected respective region 362.

In another example, the processor displays only electrograms in the regions that are instantaneous signals simultaneously captured with a multi-electrode catheter. In this example, the electrograms are listed based on the electrode number from which they originate.

In both cases the user or a processor running an algorithm perform a spatiotemporal analysis to identify a pattern of propagation cardiac activation in regions 362. As seen, Fig. 3 ties (374, 376) regions 362 to the respective subsets (323, 333) of intracardiac electrograms (321, 331), the subsets (323, 333) graphically indicated by arrows (325, 345) to assess the spatiotemporal progression of the focal and rotor arrhythmia.

### METHOD TO VISUALLY CONNECT FEATURES IDENTIFIED IN THE EA MAP TO ECG SIGNALS

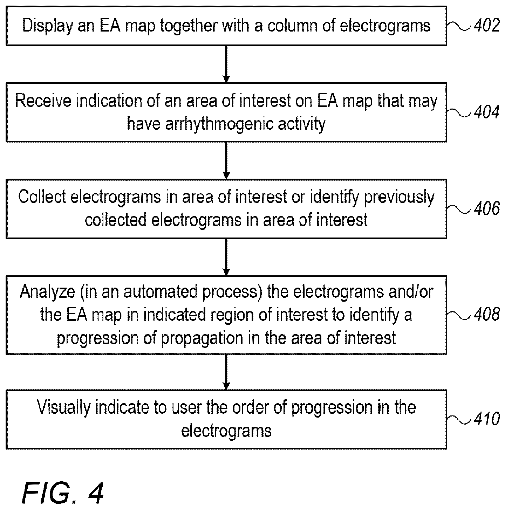

Fig. 4 is a flow chart that schematically illustrates a method to tie a graphical indication of a regional arrhythmogenic activity on an EA map to a subset of intracardiac electrograms and to collectively graphically indicate arrhythmogenic characteristics on the subset, in accordance with an example of the present disclosure.

The process carries out an algorithm that begins with a processor presenting, on a display service, an EA map of at least a portion of a cardiac chamber that may include arrhythmogenic activity together with a column of electrograms, at an EA map and related electrograms displaying step 402.

In an arrhythmogenic activity indication step 404, either the processor, or a user receive indication of an area of interest that may have arrhythmogenic activity.

In electrogram collection step 406, the processor collects electrograms in the area of interest or identify previously collected electrograms in the area of interest. Optionally, the system substantially simultaneously samples electrograms from electrodes at a distal end of the catheter. The distal end may include for example 20 - 120 electrodes each capturing a electrograms at a different location. Location at which each electrogram may be known. For example, position and orientation of a distal end of a catheter shaft may be monitored based on a magnetic based position sensor and the location of each of the electrodes may be inferred based on their known location with respect to the shaft. Optionally, an impedance based tracking may provide indication of location of the electrodes.

At an analysis step 408, the processor analyzes (in an automated process) the electrograms and/or the EA map in that area to identify a progression of the propagation in the area of interest. Optionally, the processor is configured to run an algorithm that identifies the arrhythmia-characterizing subset of electrograms among the entire set. An algorithm to find the subset typically uses some spatiotemporal and/or amplitude characteristics displayed by the relevant electrograms and an order of a propagation signal within the subset.

Identification of the arrhythmogenic propagation (e.g., one of a rotor or a focal type pattern of propagation) may be based on analysis of the electrograms, analysis of one or more EA maps or analysis of both the electrograms and the one or more EA maps. In some example an order at which an activation signal appeared in each of the electrograms in the column of electrograms is determined and this information is related to the relative location at which each electrograms was captured.

Finally, an a visual indication step 410, the processor visually indicates to the user the order of progression in the electrograms, as seen by the arrows (225, 245) in Figs 2A and 2B and arrows (325, 345) in Fig. 3, respectively.

### EXAMPLES

### Example 1

A system (10) includes a display device (27) and a processor (56). The processor (56) is configured to (i) present, on the display device (27), intracardiac electrograms (221, 231) recorded over tissue of a portion of a cardiac chamber, (ii) identify a subset (223, 233) of the electrograms (221, 231) that show arrhythmogenic activity, (iii) analyze one or more characteristics of the arrhythmogenic activity, and (iv) graphically interconnect (225, 245) the electrograms in the subset (223, 233) to present the one or more characteristics of the arrhythmogenic activity to a user.

### Example 2

The system (10) according to example 1, wherein the one or more characteristics comprise at least one of activation amplitudes, activation timings, spatiotemporal progression of activation, and activation patterns.

### Example 3

The system (10) according to any of examples 1 and 2, wherein the processor (56) is configured to graphically interconnect (225, 245) the electrograms of the subset (223, 233) to present the one or more characteristics by using arrows.

### Example 4

The system (10) according to any of examples 1 through 3, wherein the processor (56) is configured to identify the subset (223, 233) of the electrograms (221, 231) by using an electroanatomical (EA) map (201, 202) of the portion of the cardiac chamber.

### Example 5

The system (10) according to any of examples 1 through 4, wherein the processor (56) is further configured to present on the display device the EA map (201, 202), to graphically indicate (215, 235) the type of the arrhythmogenic activity on the EA map, and to graphically connect (227, 237) the activity on the EA map (201, 202) to at least one of the electrograms in the subset (223, 233) .

### Example 6

The system (10) according to any of examples 1 through 5, wherein the processor (56) is configured to graphically interconnect at least some of the electrograms in the subset (223, 233) by basing on a spatiotemporal relation between arrhythmogenic activations in the electrograms of the subset (223, 233).

### Example 7

The system (10) according to any of examples 1 through 6, wherein the processor (56) is further configured to annotate arrhythmogenic activations of the arrhythmogenic activity over at least one of the electrograms in the subset (223, 233).

### Example 8

8. The system (10) according to any of examples 1 through 7, wherein the processor (56) is further configured to present at least some of the electrograms in the subset (223, 233) on the display device (27) in an order that is based on a spatiotemporal progression of the arrhythmogenic activity.

### Example 9

The system (10) according to any of examples 1 through 8, wherein the processor (56) is further configured to identify on the EA map (201, 202) one of a regional focal source and rotor types of arrhythmogenic activity.

### Example 10

10. The system (10) according to any of examples 1 through 9, wherein the processor (56) is further configured to present the type of the arrhythmogenic activity on the EA map by (201, 202) presenting (215, 235) one of a regional focal source and rotor types of arrhythmogenic activity.

### Example 11

A method includes presenting on a display device (27) intracardiac electrograms (221, 231) recorded over tissue of a portion of a cardiac chamber. A subset (223, 233) of the electrograms is identified that show arrhythmogenic activity. One or more characteristics of the arrhythmogenic activity are analyzed. The electrograms in the subset (223, 233) are graphically interconnected (225, 245) to present the one or more characteristics of the arrhythmogenic activity to a user.

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A system (10), comprising:
a display device (27); and
a processor (56), which is configured to:
present, on the display device (27), intracardiac electrograms (221, 231) recorded over tissue of a portion of a cardiac chamber;
identify a subset (223, 233) of the electrograms (221, 231) that show arrhythmogenic activity;
analyze one or more characteristics of the arrhythmogenic activity; and
graphically interconnect (225, 245) the electrograms in the subset (223, 233) to present the one or more characteristics of the arrhythmogenic activity to a user.

2. The system (10) according to claim 1, wherein the one or more characteristics comprise at least one of activation amplitudes, activation timings, spatiotemporal progression of activation, and activation patterns.

3. The system (10) according to claim 1 or claim 2, wherein the processor (56) is configured to graphically interconnect (225, 245) the electrograms of the subset (223, 233) to present the one or more characteristics by using arrows.

4. The system (10) according to any preceding claim, wherein the processor (56) is configured to identify the subset (223, 233) of the electrograms (221, 231) by using an electroanatomical (EA) map (201, 202) of the portion of the cardiac chamber, optionally wherein the processor (56) is further configured to present on the display device the EA map (201, 202), to graphically indicate (215, 235) the type of the arrhythmogenic activity on the EA map, and to graphically connect (227, 237) the activity on the EA map (201, 202) to at least one of the electrograms in the subset (223, 233).

5. The system (10) according to any preceding claim, wherein the processor (56) is configured to graphically interconnect at least some of the electrograms in the subset (223, 233) by basing on a spatiotemporal relation between arrhythmogenic activations in the electrograms of the subset (223, 233).

6. The system (10) according to any preceding claim, wherein the processor (56) is further configured to annotate arrhythmogenic activations of the arrhythmogenic activity over at least one of the electrograms in the subset (223, 233).

7. The system (10) according to any preceding claim, wherein the processor (56) is further configured to present at least some of the electrograms in the subset (223, 233) on the display device (27) in an order that is based on a spatiotemporal progression of the arrhythmogenic activity.

8. The system (10) according to any preceding claim, wherein the processor (56) is further configured to identify on the EA map (201, 202) one of a regional focal source and rotor types of arrhythmogenic activity.

9. The system (10) according to any preceding claim, wherein the processor (56) is further configured to present the type of the arrhythmogenic activity on the EA map by (201, 202) presenting (215, 235) one of a regional focal source and rotor types of arrhythmogenic activity.

10. A method, comprising:
presenting on a display device (27) intracardiac electrograms (221, 231) recorded over tissue of a portion of a cardiac chamber;
identifying a subset (223, 233) of the electrograms that show arrhythmogenic activity;
analyzing one or more characteristics of the arrhythmogenic activity; and
graphically interconnecting (225, 245) the electrograms in the subset (223, 233) to present the one or more characteristics of the arrhythmogenic activity to a user.

11. The method according to claim 10, wherein the one or more characteristics comprise at least one of activation amplitudes, activation timings, spatiotemporal progression of activation, and activation patterns.

12. The method according to claim 10 or claim 11, wherein graphically interconnecting (225, 245) the electrograms of the subset (223, 233) to present the one or more characteristics comprises using arrows.

13. The method according to any of claims 10, to 12 wherein identifying the subset (223, 233) of the electrograms (221, 231) comprises using an electroanatomical (EA) map (201, 202) of the portion of the cardiac chamber, and optionally comprising presenting on the display device (27) the EA map (201, 202), graphically indicating (215, 235) the type of the arrhythmogenic activity on the EA map, and graphically connecting (227, 237) the activity on the EA map (201, 202) to at least one of the electrograms in the subset (223, 233) .

14. The method according to any of claims 10 to 13, graphically interconnecting at least some of the electrograms in the subset (223, 233) comprises basing on a spatiotemporal relation between arrhythmogenic activations in the electrograms of the subset (223, 233).

15. The method according to any of claims 10 to 14, and comprising annotating arrhythmogenic activations of the arrhythmogenic activity over at least one of the electrograms in the subset (223, 233).

16. The method according to any of claims 10 to 15, and comprising presenting at least some of the electrograms in the subset (223, 233) on the display device (27) in an order that is based on a spatiotemporal progression of the arrhythmogenic activity.

17. The method according to any of claims 10 to 16, and comprising identifying on the EA map (201, 202) one of a regional focal source and rotor types of arrhythmogenic activity.

18. The system according to any of claims 10 to 17, and comprising presenting the type of the arrhythmogenic activity on the EA map by (201, 202) presenting (215, 235) one of a regional focal source and rotor types of arrhythmogenic activity.
